# EUROPÄISCHE PATENTSCHRIFT

(11) **EP 0 453 393 B1**
(45) Veröffentlichungstag und Bekanntmachung des Hinweises auf die Patenterteilung: **06.10.1993**
(21) Anmeldenummer: 91810173.4
(22) Anmeldetag: 15.03.1991
(51) Int. Cl.: A61F 2/44

(54) **Implantat, insbesondere Zwischenwirbelprothese**
Implant, particularly intervertebral prosthesis
Implant, en particulier prothèse intervertébrale

(30) Priorität: 20.04.1990 CH 1324/90
(43) Veröffentlichungstag der Anmeldung: 23.10.1991
(73) Patentinhaber: SULZER Medizinaltechnik AG, 8404 Winterthur (CH)
(72) Erfinder: Baumgartner, Walter, CH-9500 Wil (CH)
(74) Vertreter: Hammer, Bruno, Dr.

(56) Entgegenhaltungen:
- EP-A- 0 260 080
- EP-A- 0 277 282
- US-A- 3 875 595
- US-A- 3 883 902

## Beschreibung

Die Erfindung betrifft ein Implantat, insbesondere eine Zwischenwirbelprothese, bestehend aus einem elastischen, flüssigkeitsdichten Hohlkörper, der mit einem inkompressiblen, fliessfähigen Medium gefüllt ist, sowie ein Instrument zum Einführen, Aufwickeln und Füllen des Implantates.

Eine Zwischenwirbelprothese der vorstehend genannten Art ist beispielsweise aus der US-A- 3,875,595 bekannt. Bei dieser Konstruktion wird ein beschädigter Bandscheibenkern (nucleus pulposus) durch ein blasenartiges Kissen aus elastischem Material ersetzt, das mit einem inkompressiblen Medium gefüllt werden kann. Dabei bleibt der äussere Ring (anulus fibrosus) einer natürlichen Bandscheibe weitgehend erhalten. Darüberhinaus ist ein rohrförmiges Instrument vorgesehen, durch das hindurch das aufblasbare Implantat zunächst im nicht gefüllten Zustand implantiert, in den benachbarten Wirbeln "verankert" und anschliessend gefüllt wird. Bei dieser Konstruktion muss der anulus fibrosus die bei einer Belastung im Nucleus erzeugten Druckkräfte als radiale Kräfte vollständig aufnehmen.

In der EP-A-0 227 282 wird eine Zwischenwirbelprothese gezeigt und beschrieben, die ebenfalls aus einem hohlen Kissen besteht, das mit einem nicht kompressiblen Medium gefüllt ist. Diese Prothese ist auf ihren, den Wirbeln zugewandten Oberflächen mit einer Struktur versehen, in die zu ihrer Fixierung Knochengewebe einwächst. Durch sie wird die ganze Bandscheibe ersetzt, wofür eine relativ weite Oeffnung zwischen den Wirbeln von ventral her freigelegt werden muss, da diese Prothese im bereits gefüllten Zustand implantiert wird.

In der EP-A-0 176 728 wird eine Zwischenwirbelprothese beschrieben, die mit dem Gleitkörper ein fixes Drehzentrum definiert, was erwiesenermassen nicht den Tatsachen bei Bewegungsvorgängen in der Wirbelsäule entspricht. Dazu werden die Deckplatten in erhöhtem Masse belastet, was zu einem Einsinken des Implantates führen kann.

Aufgabe der Erfindung ist es, ein Implantat als Ersatz für den Nucleus einer Bandscheibe zu schaffen, bei dem die Nachteile der vorstehend geschilderten Konstruktionen vermieden sind; das zu schaffende Implantat soll dabei einerseits die radialen Kräfte, die über seine inkompressible Füllung auf seine Peripherie ausgeübt werden, aufnehmen, andererseits jedoch nur einen relativ geringfügigen Eingriff in den Zwischenwirbelbereich mit einer möglichst kleinen Oeffnung erfordern.

Diese Aufgabe wird mit der Erfindung dadurch gelöst, dass der Hohlkörper aus einer langgestreckten, flexiblen Kammer besteht, an deren einem Ende ein um eine Achse drehbares Ventil vorhanden ist, dass die Kammer ferner auf das Ventil aufwickelbar ist, und dass sich schliesslich das andere Ende der Kammer in einem kompakten Band fortsetzt.

Mit dem Ventil voraus, über das eine Füllung nach dem Implantieren der Kammer erfolgt, wird die neue Konstruktion in den vom zuvor entfernten nucleus pulposus eingenommenen Raum eingeführt und durch Rotation des Ventils auf dieses aufgewickelt, ehe die Kammer gefüllt wird. Wie die Prothese nach dem US-Patent 3,875,595 kann das neue Implantat daher durch ein einfaches Rohr hindurch von dorsal implantiert werden, wofür ein durch dieses Rohr einführbares Instrument zum Einführen, Aufwickeln und Füllen des Implantes entwickelt worden ist, das seinerseits dadurch gekennzeichnet ist, dass es ein rohrartiges Führungsstück mit zwei axialen Kanälen aufweist, von denen einer für die Zufuhr des Füllmediums bestimmt ist, dass ferner das Führungsstück eine Einrichtung für einen lösbaren Anschluss an das Ventil aufweist, und dass schliesslich der zweite Kanal den Rotationsantrieb für den drehbaren Ventilteil enthält.

Die geforderte Fähigkeit, die radialen Kräfte aufzunehmen, wird durch das an die Kammer anschliessende kompakte Band erreicht, das die Kammer nach der Implantation mehrfach umschlingt und mit seinem Ende an diesen Umschlingungen fixiert ist.

Die neue Konstruktion weist darüberhinaus keine fixierten Drehzentren auf. Weiterhin sind die Deckplatten wie beim natürlichen nucleus pulposus belastet. Schliesslich wird die Funktion des anulus fibrosus durch die neue Konstruktion unterstützt.

Mit Vorteil bestehen die Kammer und das Band aus einem Stück und aus einer textilen Struktur - d.h. beispielsweise einem Gewebe, Gewirk, Gestrick oder Geflecht -, die teilweise von einem Elastomer umhüllt ist, um Abrieb der Textilstruktur zu vermeiden. Weiterhin hat es sich als zweckmässig erwiesen, wenn die parallel zur Drehachse des Ventils verlaufende Höhe der Kammer grösser ist als ihre Breite, wobei der querschnitt beispielsweise mindestens nahezu rechteckig sein kann; durch diese beiden Merkmale ergibt sich ein Wickelkörper, dessen Windungen mit relativ grossen glatten Auflageflächen aneinander liegen, wobei sich beim Aufwickeln eine Anzahl Windungen bildet, die ihrerseits eine Anpassung an die Form des Hohlraumes, in die die Prothese eingelegt wird, erleichtern. Diese Anpassung kann noch weiter unterstützt werden, wenn die Höhe der Kammer variabel ist.

Für das an einem Ende der Kammer angesetzte Ventil hat sich eine Konstruktion bewährt, die eine feststehende Basis mit einem ersten Rohrabschnitt aufweist, in dem ein Ventilsitz und ein Ventilkörper vorhanden sind, und von dem ein zweiter Rohrabschnitt, der als feststehender Lagerzapfen für einen drehbaren Aufwickelkörper dient, in die Kammer führt, deren eines Ende am Aufwickelkörper befestigt ist; der Aufwickelkörper kann dabei zusätzlich mit einem Zahnkranz für den Eingriff des Antriebs des Instrumentes versehen sein.

Bei diesem ist es zweckmässig, wenn die Einrichtung für den Anschluss des Ventilteils aus in den feststehenden Ventilteil eingreifenden Zapfen besteht, die aus der Stirnseite des Führungsstücks herausragen; weiterhin ist der Antrieb für die Rotation des Ventilteils vorteilhafterweise ein geschlossener Riemen mit einer geriffelten Oberfläche, deren Riffelung in den Zahnkranz des Aufwickelkörpers eingreift.

Im folgenden wird die Erfindung anhand eines Ausführungsbeispiels im Zusammenhang mit der Zeichnung näher erläutert.
- Fig. 1: zeigt schematisch den Hohlkörper des Implantates in abgewickelter Form;
- Fig. 2 - 4: sind Schnitt II-II bis IV-IV von Fig. 1;
- Fig. 5: gibt, ebenfalls in schematischer Darstellung, das Einführinstrument wieder;
- Fig. 6: zeigt schematisch das Einführinstrument mit daran angesetztem Ventil und Hohlkörper in dem Rohr, durch das hindurch die Implantation erfolgt;
- Fig. 7: stellt in einer Aufsicht auf einen Wirbelkörper das eingesetzte, aufgewickelte und gefüllte Implantat dar, während
- Fig. 8: ein Schnitt VIII-VIII von Fig. 7 ist.

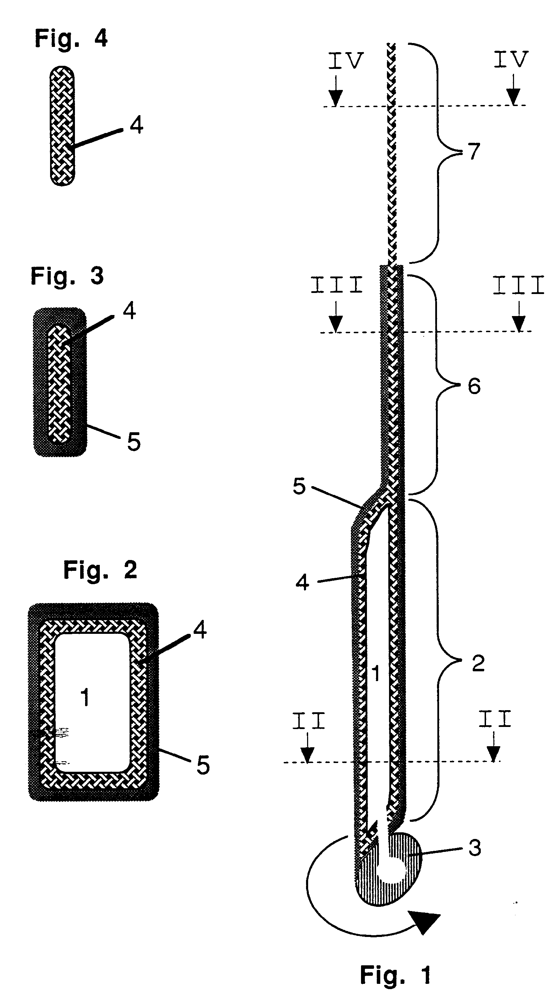

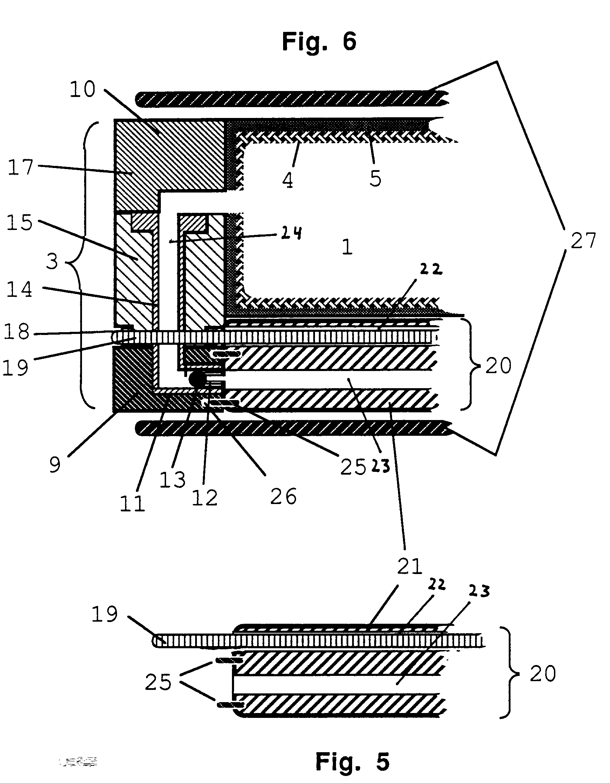

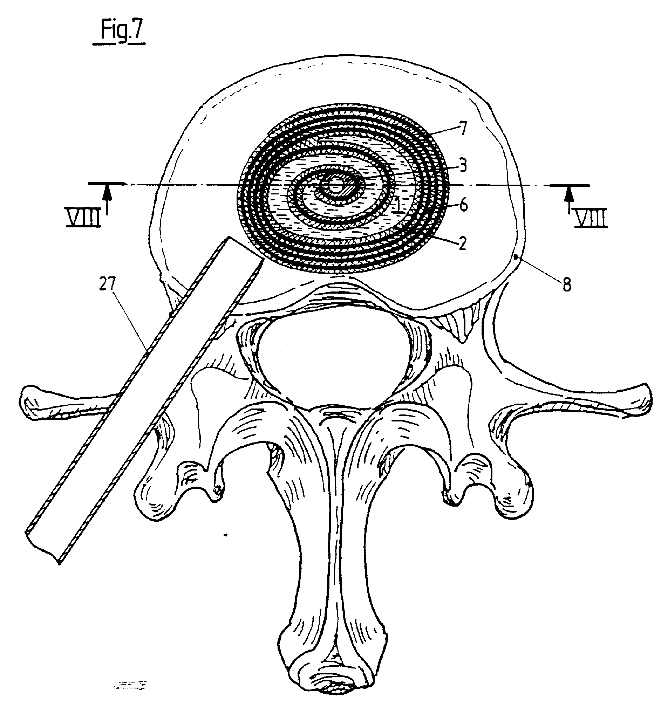

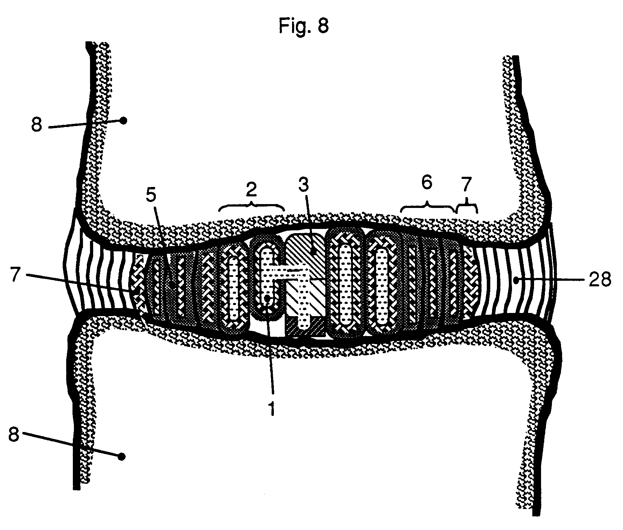

Ein Hohlraum oder eine Kammer 1 (Fig. 1) eines bandartigen Hohlkörpers 2 ist an einem Ende mit einem in Fig. 1 nur schematisch dargestellten Ventil 3 verbunden, dessen Aufbau später beschrieben wird. Der flexible Hohlkörper (Fig. 2) besteht aus einem, aus Kunststoff-Fäden, beispielsweise Polyethylen Terephthalat, gefertigten textilen Maschenwerk 4, das aussen mit einer Elastomerschicht 5, beispielsweise einem Polyurethan, mittels eines Tauchverfahrens beschichtet ist. Die Polyurethanschicht hat die Aufgabe, ein Reiben der Textilfäden aufeinander bei eingesetztem Implantat zu verhindern. Wie Fig. 1 zeigt, setzt sich die Kammer in einem kompakten textilen Band 6 fort (Fig. 3), das über eine gewisse Länge ebenfalls noch mit dem Elastomer 5 beschichtet ist, an seinem freien Ende 7 (Fig. 4) jedoch keine Beschichtung 5 mehr trägt. Das freie Ende 7 kann somit relativ einfach durch Vernähen oder Verschweissen bei einer zwischen zwei Wirbeln 8 (Fig. 7 und 8) implantierten Prothese auf sich selbst fixiert werden.

Das in Fig. 6 gezeigte Ventil 3 setzt sich aus einem feststehenden Ventilteil 9 und einem drehbaren Aufwickelkörper 10 zusammen. Beide Teile bestehen beispielsweise aus einem körperfreundlichen Metall, wie Titan oder eine Titanlegierung. Der feststehende Ventilteil 9 enthält einen, beispielsweise aus rostfreiem Stahl bestehenden ersten Rohrabschnitt 11, in den ein metallener Ventilsitz 12 und als Verschlusskörper beispielsweise eine Metallkugel 13 eingebaut sind.

An den Rohrabschnitt 11 schliesst sich ein zweiter Rohrabschnitt 14 an, der in die Kammer 1 des Hohlkörpers 2 führt. Er dient gleichzeitig als feststehender Lagerzapfen für den drehbaren Aufwickelkörper 10. Dieser ist aus Montagegründen unterteilt in einen den Rohrabschnitt 14 umgebenden Unterteil 15 und einen oberen Teil 17. An seinem Boden ist der Unterteil 15 als Zahnkranz 18 ausgebildet, in den als Rotationsantrieb ein Antriebsriemen 19 eingreift, der beispielsweise aus einem glasfaserverstärktem Kunststoff besteht.

Der Antriebsriemen 19 ist ein Teil eines in Fig. 5 gezeigten Instrumentes 20 zum Einführen, Aufwickeln und Füllen des Implantates. Das Instrument 20 besteht in vorliegenden Beispiel im wesentlichen aus einem metallenen Führungsstück 21, in dem zwei Längsbohrungen oder Kanäle 22 und 23 vorgesehen sind. Die eine 22 dieser Bohrungen nimmt den Antriebsriemen 19 auf, während die andere 23, die an den Rohrabschnitt 11 des Ventilteils 9 anschliessbar ist, für die Zuführung des inkompressiblen Füllmittels, beispielsweise Hydroxin-Ethyl-Methacrylat, in die Kammer 1 vorgesehen ist; das Füllmittel fliesst dabei durch das aus Ventilsitz 12 und Verschlusskörper 13 bestehende Rückschlagventil über den Strömungsweg 24 in den Rohrabschnitten 11 und 14 und dem Oberteil 17 der Kammer 1 zu.

Für eine starre, aber lösbare Verbindung des Führungsstücks 21 mit dem Ventilteil 9 sind in der Stirnsteite des Führungsstücks 21 zwei Zapfen 25 vorgesehen, die in entsprechende Ausnehmungen 26 im Ventilteil 9 einsteckbar sind.

Das Implantat zusammen mit seinem Führungsinstrument 20 ist für die Implantation in einem Rohr 27 untergebracht.

Mit 28 ist in Fig. 8 das natürliche Gewebe des anulus fibrosus bezeichnet, das das Implantat 1 umschliesst.

Das Einsetzen des Implantates zwischen zwei Wirbel 8 kann beispielsweise ähnlich wie bei dem eingangs erwähnten Implantat nach dem US-Patent 3,875,595 von dorsal her durch das Rohr 27 hindurch erfolgen, für dessen Einführung zwischen die Wirbel nur ein relativ geringfügiger Eingriff notwendig ist. Nach dem "Setzen"'des Rohres 27 werden der nucleus pulposus und gegebenenfalls schadhafte Teile des anulus fibrosus entfernt und anschliessend das Implantat, das zuvor mit dem Instrument 20 starr verbunden worden ist, durch das Rohr 27 in den entstandenen Hohlraum eingeführt. Nachdem das Ventil 3 in die gewünschte Lage gebracht worden ist, wird der bandartige Teil 2, 6, 7 des Implantates durch Ziehen an einer Seite des als geschlossener Ring ausgebildeten Antriebsriemens 19 auf das Ventil aufgewickelt. Nach Beendigung des Wickelvorganges wird das freie Ende 7 des Bandes durch Schweissen oder Nähen auf sich selbst fixiert und der Riemen 19 aufgetrennt und entfernt. Nunmehr wird die Kammer 1 durch die Bohrung 23 im Führungstück 21 hindurch mit dem Füllmedium gefüllt. Ist die gewünschte Menge Füllmittel eingefüllt, so wird das Führungsstück 21 vom Ventilteil 9 gelöst und durch das Rohr 27 entfernt. Abschliessend entfernt man noch das Rohr 27 aus dem Körper.

## Patentansprüche

1. Implantat, insbesondere Zwischenwirbelprothese, bestehend aus einem elastischen, flüssigkeitsdichten Hohlkörper (2), der mit einem inkompressiblen, fliessfähigen Medium füllbar ist, dadurch gekennzeichnet, dass der Hohlkörper (2) aus einer langgestreckten, flexiblen Kammer (1) besteht, an deren einem Ende ein um eine Achse drehbares Ventil (3) vorhanden ist, dass die Kammer (1) ferner auf das Ventil (3) aufwickelbar ist, und dass sich schliesslich das andere Ende der Kammer (1) in einem kompakten Band (6,7) fortsetzt.

2. Implantat nach Anspruch 1, dadurch gekennzeichnet, dass die Kammer (1) und das Band (6,7) aus einer textilen Struktur (4) bestehen, die mindestens auf einer Teillänge von einem Elastomer (5) umhüllt ist.

3. Implantat nach Anspruch 1 oder 2, dadurch gekennzeichnet, dass die parallel zur Drehachse des Ventils (3) verlaufende Höhe der Kammer (1) grösser ist als ihre Breite.

4. Implantat nach Anspruch 3 dadurch gekennzeichnet, dass der Querschnitt der Kammer (1) rechteckig ist.

5. Implantat nach Anspruch 3 oder 4, dadurch gekennzeichnet, dass die Höhe der Kammer (1) variabel ist.

6. Implantat nach einem der Ansprüche 1 bis 5, dadurch gekennzeichnet, dass das Ventil (3) eine feststehende Basis (9) mit einem ersten Rohrabschnitt (11) aufweist, in dem ein Ventilsitz (12) und ein Verschlusskörper (13) vorhanden sind, und von dem ein zweiter Rohrabschnitt (14), der als feststehender Lagerzapfen für einen drehbaren Aufwickelkörper (10) dient, in die Kammer (1) führt, deren eines Ende am Aufwickelkörper (10) befestigt ist.

7. Implantat nach Anspruch 6, dadurch gekennzeichnet, dass der Aufwickelkörper (10) mit einem Zahnkranz (18) für den Eingriff eines Antriebsriemens (19) eines Instrumentes (20) versehen ist.

8. Instrument zum Einführen, Aufwickeln und Füllen des Implantates nach einem der Ansprüche 1 bis 7, dadurch gekennzeichnet, dass das Instrument (20) ein rohrartiges Führungsstück (21) mit zwei axialen Kanälen (22, 23) aufweist, von denen einer (23) für die Zufuhr des Füllmediums bestimmt ist, dass ferner das Führungsstück (21) eine Einrichtung (25, 26) für einen lösbaren Anschluss an das Ventil (3) aufweist, und dass schliesslich der zweite Kanal (22) den Rotationsantrieb für den drehbaren Ventilteil (10) enthält.

9. Instrument nach Anspruch 8, dadurch gekennzeichnet, dass die Einrichtung (25, 26) für den Anschluss des Ventilteiles (10) aus in den feststehenden Ventilteil (9) eingreifenden Zapfen (25) besteht, die aus der Stirnseite des Führungsstücks (21) herausragen.

10. Instrument nach Anspruch 8 oder 9, dadurch gekennzeichnet, dass der Antrieb (19) für die Rotation des Ventilteiles (10) ein geschlossener Riemen (19) mit einer geriffelten Oberfläche ist, deren Riffelung in einen Zahnkranz (18) des Aufwickelkörpers (10) eingreift.

## Claims

1. An implant, especially an intervertebral prosthesis, consisting of an elastic liquidtight hollow body (2) which may be filled with an incompressible free-flowing medium, characterized in that the hollow body (2) consists of an elongated flexible chamber (1) at one end of which there is a valve (3) which may be turned about an axis, that the chamber (1) may further be coiled up round the valve (3) and that finally the other end of the chamber (1) continues into a compact strip (6, 7).

2. An implant as in Claim 1, characterized in that the chamber (1) and strip (6, 7) consist of a textile structure (4) which over at least part of its length is enveloped in an elastomer (5).

3. An implant as in Claim 1 or 2, characterized in that the height of the chamber (1) running in parallel with the axis of rotation of the valve (3) is greater than its width.

4. An implant as in Claim 3, characterized in that the cross-section of the chamber (1) is rectangular.

5. An implant as in Claim 3 or 4, characterized in that the height of the chamber (1) is variable.

6. An implant as in one of the Claims 1 to 5, characterized in that the valve (3) exhibits a fixed base (9) having a first tubular portion (11) in which there are a valve seat (12) and a means of closure (13) and from which a second tubular portion (14) which serves as a fixed pivot for a turnable coiler body (10) leads into the chamber (1) of which one end is fastened to the coiler body (10).

7. An implant as in Claim 6, characterized in that the coller body (10) is provided with a toothed ring (18) for engagement with a driving belt (19) from an instrument (20).

8. An instrument for the introduction, coiling up and filling of the implant as in one of the Claims 1 to 7, characterized in that the instrument (20) exhibits a tubular guidepiece (21) having two axial channels (22, 23) of which one (23) is intended for feeding in the filling medium, that further the guidepiece (21) exhibits a device (25, 26) for detachable connection to the valve (3), and that finally the second channel (22) contains the drive for rotation of the turnable part (10) of the valve.

9. An instrument as in Claim 8, characterized in that the device (25, 26) for connection to the part (10) of the valve consists of pins (25) engaging in the fixed part (9) of the valve, which project from the endface of the guidepiece (21).

10. An instrument as in Claim 8 or 9, characterized in that the drive (19) for rotation of the part (10) of the valve is an endless belt having a ribbed surface the ribbing on which engages in a toothed ring (18) on the coiler body (10).

## Revendications

1. Implant, en particulier prothèse inter-vertébrale, se composant d'un corps creux élastique (2) étanche aux liquides et pouvant être rempli d'un milieu incompressible et capable de fluer, caractérisé en ce que le corps creux (2) consiste en une chambre allongée et flexible (1), à une extrémité de laquelle se trouve un clapet (3) rotatif autour d'un axe, en ce que la chambre (1) est par ailleurs enroulable sur le clapet (3) et en ce que finalement l'autre extrémité de la chambre (1) se prolonge en un ruban compact (6, 7).

2. Implant selon la revendication 1, caractérisé en ce que la chambre (1) et le ruban (6, 7) se composent d'une structure textile (4) qui est enveloppée au moins sur une partie de la longueur par un élastomère (5).

3. Implant selon la revendication 1 ou 2, caractérisé en ce que la hauteur de la chambre (1) parallèlement à l'axe de rotation du clapet (3) est supérieure à sa largeur.

4. Implant selon la revendication 3, caractérisé en ce que la section de la chambre (1) est rectangulaire.

5. Implant selon la revendication 3 ou 4, caractérisé en ce que la hauteur de la chambre (1) est variable.

6. Implant selon l'une des revendications 1 à 5, caractérisé en ce que le clapet (3) comporte une base fixe (9) incluant un premier tronçon de tube (11) dans lequel se trouvent un siège (12) de clapet et un corps obturateur (13) et dont un second tronçon de tube (14), qui sert de tourillon fixe pour un corps rotatif d'enroulement (10), mène dans la chambre (1), dont une extrémité est fixée au corps d'enroulement (10).

7. Implant selon la revendication 6, caractérisé en ce que le corps d'enroulement (10) est équipé d'une couronne dentée (18) pour l'engrènement d'une courroie de commande (19) d'un instrument (20).

8. Instrument d'introduction, d'enroulement et de remplissage de l'implant selon l'une des revendications 1 à 7, caractérisé en ce que l'instrument (20) comprend une pièce tubulaire de guidage (21) comportant deux canaux axiaux (22, 23), dont l'un, (23), est destiné à l'admission du milieu de remplissage, en ce que par ailleurs la pièce de guidage (21) comprend un dispositif (25, 26) de raccord amovible au clapet (3) et en ce que finalement le second canal (22) loge la commande de rotation de l'élément rotatif de clapet (10).

9. Instrument selon la revendication 8, caractérisé en ce que le dispositif (25, 26) de raccord de l'élément de clapet (10) se compose de tétons (25) se logeant dans l'élément fixe de clapet (9) et qui sont saillants sur le côté extrême de la pièce de guidage (21).

10. Instrument selon la revendication 8 ou 9, caractérisé en ce que la commande (19) de la rotation de l'élément de clapet (10) est une courroie fermée (19) à surface crantée, dont le crantage engrène avec une couronne dentée (18) du corps d'enroulement (10).
